(19) Europäisches Patentamt
European Patent Office
Office européen des brevets

(11) **EP 2 030 592 B1**

(12) **EUROPEAN PATENT SPECIFICATION**

(45) Date of publication and mention
of the grant of the patent:
**05.05.2010  Bulletin 2010/18**

(51) Int Cl.:
***A61F 2/06*** (2006.01)

(21) Application number: **08171168.1**

(22) Date of filing: **02.08.2007**

(54) **Stent graft sealing zone connecting structure**

Verbindungsstruktur für den Versiegelungsbereich eines Stentimplantats

Structure de raccordement de zone de scellement de greffe d'endoprothèse

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **11.08.2006  US 503362**

(43) Date of publication of application:
**04.03.2009  Bulletin 2009/10**

(62) Document number(s) of the earlier application(s) in accordance with Art. 76 EPC:
**07253049.6 / 1 886 645**

(73) Proprietor: **Cordis Corporation**
**Miami Lakes, FL 33014 (US)**

(72) Inventors:
• **Majercak, David C**
**Stewartsville, NJ NJ 08886 (US)**
• **Park, Jin S.**
**Parsippany, NJ 07054 (US)**

(74) Representative: **Brown, George Laurence**
**Urquhart-Dykes & Lord LLP**
**Tower North Central**
**Merrion Way**
**Leeds LS2 8PA (GB)**

(56) References cited:
**EP-A- 0 480 667     WO-A-00/64355**
**WO-A-96/21404**

**Description**

This invention relates broadly to surgical implants. More particularly, this invention relates to connecting structure at the sealing zone for multi-part stents particularly useful in synthetic grafts, although it is not limited thereto.

An aneurysm is an abnormal dilation of a layer or layers of an arterial wall, usually caused by a systemic collagen or structural defect. An abdominal aortic aneurysm (AAA) is an aneurysm in the abdominal portion of the aorta, usually located in or near one or both of the two iliac arteries or near the renal arteries. The aneurysm often arises in the infrarenal portion of the diseased aorta, for example, below the kidneys. A thoracic aortic aneurysm is an aneurysm in the thoracic portion of the aorta. When left untreated, the aneurysm may rupture, usually causing rapid fatal hemorrhaging.

Aneurysms may be classified or typed by their position as well as by the number of aneurysms in a cluster. Typically, abdominal aortic aneurysms may be classified into five types. A Type I aneurysm is a single dilation located between the renal arteries and the iliac arteries. Typically, in a Type I aneurysm, the aorta is healthy between the renal arteries and the aneurysm and between the aneurysm and the iliac arteries.

A Type II A aneurysm is a single dilation located between the renal arteries and the iliac arteries. In a Type II A aneurysm, the aorta is healthy between the renal arteries and the aneurysm, but not healthy between the aneurysm and the iliac arteries. In other words, the dilation extends to the aortic bifurcation. A Type II B aneurysm comprises three dilations. One dilation is located between the renal arteries and the iliac arteries. Like a Type II A aneurysm, the aorta is healthy between the aneurysm and the renal arteries, but not healthy between the aneurysm and the iliac arteries. The other two dilations are located in the iliac arteries between the aortic bifurcation and the bifurcations between the external iliacs and the internal iliacs. The iliac arteries are healthy between the iliac bifurcation and the aneurysms. A Type II C aneurysm also comprises three dilations. However, in a Type II C aneurysm, the dilations in the iliac arteries extend to the iliac bifurcation.

A Type III aneurysm is a single dilation located between the renal arteries and the iliac arteries. In a Type III aneurysm, the aorta is not healthy between the renal arteries and the aneurysm. In other words, the dilation extends to the renal arteries.

A ruptured abdominal aortic aneurysm is presently the thirteenth leading cause of death in the United States. The routine management of abdominal aortic aneurysms has been surgical bypass, with the placement of a graft in the involved or dilated segment. Although resection with a synthetic graft via a transperitoneal or retroperitoneal procedure has been the standard treatment, it is associated with significant risk. For example, complications include perioperative myocardial ischemia, renal failure, erectile impotence, intestinal ischemia, infection, lower limb ischemia, spinal cord injury with paralysis, aorta-enteric fistula, and death. Surgical treatment of abdominal aortic aneurysms is associated with an overall mortality rate of five percent in asymptomatic patients, sixteen to nineteen percent in symptomatic patients, and is as high as fifty percent in patients with ruptured abdominal aortic aneurysms.

Disadvantages associated with conventional surgery, in addition to the high mortality rate, include an extended recovery period associated with the large surgical incision and the opening of the abdominal cavity, difficulties in suturing the graft to the aorta, the loss of the existing thrombosis to support and reinforce the graft, the unsuitability of the surgery for many patients having abdominal aortic aneurysms, and the problems associated with performing the surgery on an emergency basis after the aneurysm has ruptured. Further, the typical recovery period is from one to two weeks in the hospital and a convalescence period, at home, ranging from two to three months or more, if complications ensue. Since many patients having abdominal aortic aneurysms have other chronic illnesses, such as heart, lung, liver and/or kidney disease, coupled with the fact that many of these patients are older, they are less than ideal candidates for surgery.

The occurrence of aneurysms is not confined to the abdominal region. While abdominal aortic aneurysms are generally the most common, aneurysms in other regions of the aorta or one of its branches are possible. For example, aneurysms may occur in the thoracic aorta. As is the case with abdominal aortic aneurysms, the widely accepted approach to treating an aneurysm in the thoracic aorta is surgical repair, involving replacing the aneurysmal segment with a prosthetic device. This surgery, as described above, is a major undertaking, with associated high risks and with significant mortality and morbidity.

Over the past five years, there has been a great deal of research directed at developing less invasive, endovascular, i.e., catheter directed, techniques for the treatment of aneurysms, specifically abdominal aortic aneurysms. This has been facilitated by the development of vascular stents, which can and have been used in conjunction with standard or thin-wall graft material in order to create a stent-graft or endograft. The potential advantages of less invasive treatments have included reduced surgical morbidity and mortality along with shorter hospital and intensive care unit stays.

Stent-grafts or endoprostheses are now Food and Drug Administration (FDA) approved and commercially available. Their delivery procedure typically involves advanced angiographic techniques performed through vascular accesses gained via surgical cut down of a remote artery, which may include the common femoral or brachial arteries. Over a guidewire, the appropriate size introducer will be placed. The catheter and guidewire are passed through the aneurysm. Through the introducer, the stent-graft will be advanced to the appropriate position. Typical deployment of the stent-graft device requires withdrawal of an outer sheath while maintaining the position of the stent-graft with an inner-stabilizing device. Most stent-grafts are self-expanding; however, an additional angioplasty procedure, e.g., balloon angioplasty, may be required to secure the position of the stent-graft. Following the placement of the stent-graft, standard angiographic views may be obtained.

Due to the large diameter of the above-described devices, typically greater than twenty French (3F=1 mm), arteriotomy closure typically requires open surgical repair. Some procedures may require additional surgical techniques, such as hypogastric artery embolization, vessel ligation, or surgical bypass in order to adequately treat the aneurysm or to maintain blood flow to both lower extremities. Likewise, some procedures will require additional advanced catheter directed techniques, such as angioplasty, stent placement and embolization, in order to successfully exclude the aneurysm and efficiently manage leaks.

While the above-described endoprostheses represent a significant improvement over conventional surgical techniques, there is a need to improve the endoprostheses, their method of use and their applicability to varied biological conditions. Accordingly, in order to provide a safe and effective alternate means for treating aneurysms, including abdominal aortic aneurysms and thoracic aortic aneurysms, a number of difficulties associated with currently known endoprostheses and their delivery systems must be overcome. One concern with the use of endoprostheses is the prevention of endo-leaks and the disruption of the normal fluid dynamics of the vasculature. Devices using any technology should preferably be simple to position and reposition as necessary, should preferably provide an acute, fluid tight seal, and should preferably be anchored to prevent migration without interfering with normal blood flow in both the aneurysmal vessel as well as branching vessels. In addition, devices using the technology should preferably be able to be anchored, sealed, and maintained in bifurcated vessels, tortuous vessels, highly angulated vessels, partially diseased vessels, calcified vessels, odd shaped vessels, short vessels, and long vessels. In order to accomplish this, the endoprostheses should preferably be highly durable, extendable and re-configurable while maintaining acute and long-term fluid tight seals and anchoring positions.

The endoprostheses should also preferably be able to be delivered percutaneously utilizing catheters, guidewires and other devices which substantially eliminate the need for open surgical intervention. Accordingly, the diameter of the endoprostheses in the catheter is an important factor. This is especially true for aneurysms in the larger vessels, such as the thoracic aorta. In addition, the endoprostheses should preferably be percutaneously delivered and deployed such that surgical cut down is unnecessary.

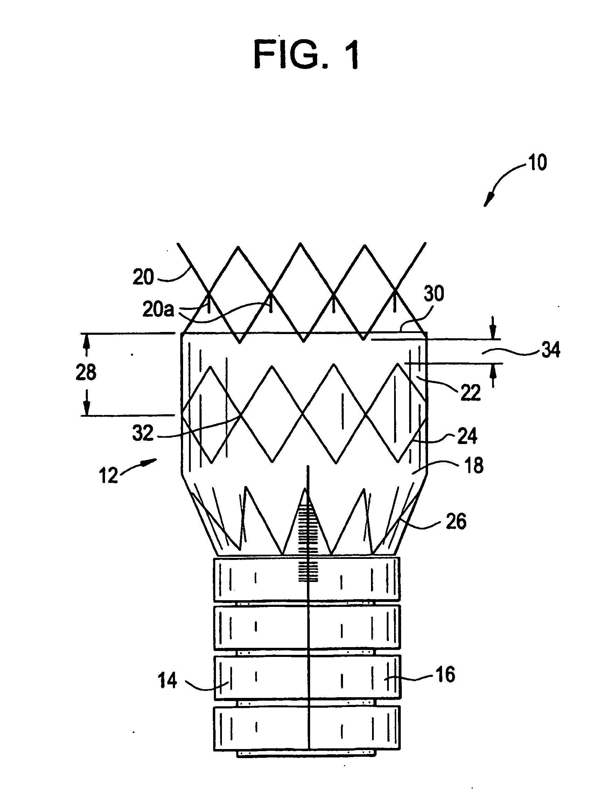

Referring to Fig. 1, a typical percutaneously delivered endoprosthesis 10 for treating an abdominal aortic aneurism is a bifurcated device having a main body 12 and two legs 14, 16. Ideally, the device lines the aorta and extends from just below the lowest renal artery into both iliac arteries up to the juncture with the hypogastric arteries. The endoprosthesis 10 is generally comprised of a fabric graft material 18 coupled to several metallic stents. The stents support the graft and hold it open within the vessels. The main body 12 may include an upper supra renal stent 20 extending above the graft material 18 and typically provided with tissue anchors 20a, one or more sealing stents 22, 24 generally extending circumferentially in a Z-shape and providing outward force against the graft to seal between the graft and the body tissue, and a tapered stent 26 that leads into the legs 14, 16.

The endoprosthesis is delivered to the aneurysm in the aorta by way of a delivery catheter. The delivery catheter containing the endoprosthesis is inserted through a small incision in the groin where it is threaded through the femoral artery and advanced to the location of the aneurysm. The surgeon uses fluoroscopy to guide the endoprosthesis and the endoprosthesis has several markers to help the surgeon visualize the graft during placement of the endoprosthesis. It is desirable that there be at least ten mm of overlap between the endoprosthesis and a healthy vessel portion. Otherwise, an opening between the two can develop that can lead to leakage.

Referring again to Fig. 1, the minimum sealing length 28, defined between the top 30 of the graft material 18 and the bottom 32 of the first sealing stent 22, is a partial determinative of how effective the endoprosthesis 10 will be in preventing or at least limiting leakage. The minimum sealing length is the length required to achieve full circumferential apposition of a portion of the stent against the vessel wall. While a longer scaling length is advantageous, the seal length must also be short enough to allow the endoprosthesis to fit the anatomy especially where the anatomy includes a severe bend.

Further, when the supra renal and sealing stents are crimped in diameter for loading and storage within a delivery device, it is recognized that due to foreshortening the effective length of the stents of the endoprosthesis will be increased. Such foreshortening could cause the stents to overlap each other, resulting in a large profile. To avoid any overlap between the supra renal and sealing stents, it is necessary to displace such stents within the graft material with a sufficiently large gap 34 so that when crimped there is a sufficient gap between the stents to avoid undesirable overlap. While avoiding overlap allows a small profile to be achieved, the minimum sealing length is compromised since the gap increases the minimum sealing length.

In addition, axial blood pressure through the endoprosthesis can create significant loading at the attachment sites, particularly between the supra renal stent and the graft material thereabout. Over time, such loading can affect the durability and integrity of the endoprosthesis.

WO 96/21404 discloses the features of the preamble of claim 1. It is therefore an object of the invention to provide an endoprosthesis with a short and effective sealing zone, such endoprosthesis particularly suitable for treatment of an abdominal aortic aneurism.

It is also an object of the invention to provide an endoprosthesis in which stent components thereof will not overlap due to foreshortening in a crimped state and in which a relatively short sealing zone is maintained, such endoprosthesis particularly suitable for treatment of an abdominal aortic aneurism.

It is another object of the invention to provide an endoprosthesis that has improved sealing capability.

It is an additional object of the invention to provide an endoprosthesis that has improved durability.

In accord with these objects, which will be discussed in detail below, an endoprosthesis, particularly adapted as an abdominal aortic aneurysm (AAA) device, is bifurcated including a main body portion and two leg portions coupled thereto. The main body portion includes a fabric graft material coupled to several metallic stent structures at attachment sites. The stent structures include an upper supra renal stent extending above the graft material, one or more sealing stents generally extending in a repeating Z-shape circumferentially within the graft and, when expanded, providing outward force against the graft, and a tapered stent that leads into the legs.

According to the present invention, there is provided an endoluminal prosthesis as defined in appended claim 1.

According to one preferred aspect of the invention, the sealing length between the top of the graft material and the bottom of the uppermost sealing stent is minimized while avoiding stent overlap or otherwise compromising the crimped profile. The invention includes connecting the apexes of the supra renal and sealing stents with a tether, preferably fixed in length, that is free to slide within the apexes. When the endoprosthesis is crimped and thus the stents are compressed in diameter, the tether has sufficient length to displace the supra renal and sealing stents by a sufficient gap to prevent compromising the crimped profile. When the endoprosthesis is expanded and thus the stents are increased in diameter, the tether which is free to move between the apexes, is pulled taut drawing the stents toward each other to decrease the minimum sealing length.

According to another preferred aspect of the invention, additional graft material is heat set into a pleat at the top of the graft. When the endoprosthesis is crimped, the pleat is unfolded to accommodate stent separation. After expansion of the endoprosthesis, the heat set of the pleat results in at least partial automatic re-gathering of the material into the pleat. The additional graft material at the pleat provides multiple layers of graft material to increase tissue contact. The graft material is thrombogenic. The increased thickness of the material provides better sealing.

According to yet another preferred aspect of the invention, the loading on the supra renal stent is shared between the tether and the attachment sites that couple the supra renal stent to the graft. Initially all the force is carried by the tether, with the force carried by the attachment sites only after the pleat has unfolded as far as it is able. Only thereafter is the downward force from blood flow carried by the attachment sites between the stent and graft.

According to another aspect of the present invention, there is provided an endoluminal prosthesis, comprising:

a) a tubular graft main body comprising a graft material including inner and outer surfaces and first and second ends;
b) a first stent coupled at said inner surface of said graft material at said first end and extending out thereof;
c) a second stent coupled at said inner surface of said graft material and extending completely therewithin; and
d) two tubular leg portions extending from said second end of said main body such that said prosthesis assumes a Y-configuration,

wherein said graft material is multiple layers thick at a junction of said first and second stents when said main body portion is in an expanded state.

At said junction, said graft material may be folded back on itself lengthwise to be three layers thick when said main body portion is in an expanded state.

The endoluminal prosthesis may further comprise:

a tether, wherein:

said first and second stents include respective sets of apexes which are nearest each other and said tether extends through and is freely movable within said apexes.

Additional objects and advantages of the invention will become apparent to those skilled in the art upon reference to the detailed description taken in conjunction with the provided figures.

Embodiments of the invention will now be described by way of example with reference to the accompanying drawings, in which:

Fig. 1 is an expanded view of an exemplar endoprosthesis designed by the inventors for the purpose of describing certain issues with respect to prior art endoprosthesis designs;

Fig. 2 is a side elevation of an endoprosthesis according to the invention;

Fig. 3 is an enlarged broken schematic section of an upper portion of an endoprosthesis according to the invention, shown in an expanded state;

Fig. 4 is an enlarged broken schematic section of an upper portion of an endoprosthesis according to the invention, shown in a crimped state;

Fig. 5 is graph of a the change in the gap between the supra renal and upper sealing stent as a result of increasing the diameter of the main body portion of the stent;

Fig. 6 is a partial section of an upper interior wall of an endoprosthesis according to the invention, shown in an expanded state; and

Fig. 7 is a schematic broken section view through an portion of an endoprosthesis according to the invention.

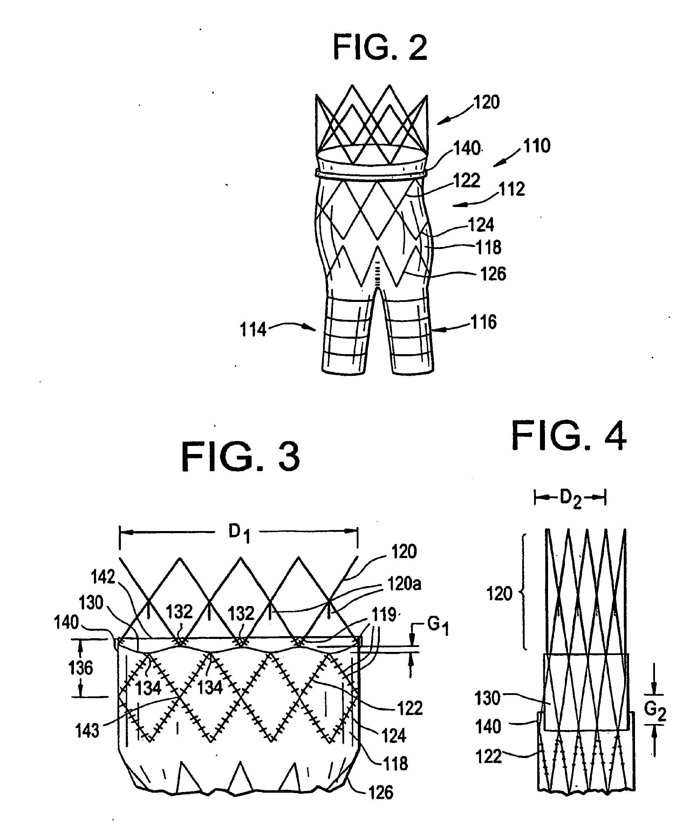

Turning now to Figs. 2 through 4, a vascular endoprosthesis 110 suitable for treatment of an abdominal aortic aneurysm (AAA) includes a main body portion 112 and two smaller leg portions 114, 116 coupled thereto in a generally inverted Y-configuration. Each portion includes graft material 118 and a plurality of expandable stents coupled at the interior of the graft material, preferably with stitching 119. The graft material is preferably made of polyethylene terephthalate (sold under the trademark Dacron®), which is thrombogenic. The stents, described in more detail below, are preferably self-expanding, formed from a superelastic, shape memory material such as Nitinol or other nickel-titanium alloy, but may be pressure expandable such as with a balloon catheter. When the stents are expanded, they provide outward force of the graft material 118 against the body tissue.

The main body portion 112 preferably includes several discrete stents: an upper supra renal stent 120 preferably extending in a circular cylindrical form (i.e., preferably non-helical) above a portion of the graft material 118 and typically provided with tissue anchors 120a, one or more sealing stents 122, 124 generally extending circumferentially about the graft in a repeating Z-shape (also preferably extending non-helically) and providing outward force against the graft to seal between the graft and the body tissue, and a tapered stent 126 that leads into the legs 114, 116.

According to one aspect of the invention, the supra renal and upper sealing stents 120, 122 are connected together with a preferably fixed length tether 130 that is free to slide within the adjacent apexes 132, 134 of such stents. The cylindrical arrangement of the stents provides that the apexes 132, 134 define points that lie on respective circles. The tether 130 is preferably comprised of Dyneema synthetic fiber available from DSM of The Netherlands, or another flexible, lubricious, high strength, high fatigue resistance, and high wear resistance material such as an HDPE fiber. When the endoprosthesis 110 is crimped and thus the stents are compressed from the expanded diameter $D_1$ (Fig. 3) to the crimped diameter $D_2$ (Fig. 4) for loading into a delivery device, the tether 130 has a length sufficient to allow the supra renal and upper sealing stents to be displaced relative to each other by a large enough gap $G_2$ to prevent compromising the crimped profile. As will be described hereinafter, a pleat 140 helps accommodate displacement of the stents 120, 122 relative to each other. When the stents are again increased in diameter to $D_2$ once the endoprosthesis is expanded, the tether 130, which is free to move between the apexes 132, 134 through which it extends, is pulled taut, drawing the stents 120, 122 toward each other to significantly reduce or even completely eliminate the gap to G1 and decrease the length of the endoprosthesis required to achieve full circumferential apposition against the vessel wall, i.e., the minimum sealing length 136 (defined as the distance between the top 142 of the graft material 118 and the bottom 143 of the first sealing stent 122) relative to prior art endoprostheses suitable for treatment of abdominal aortic aneurism.

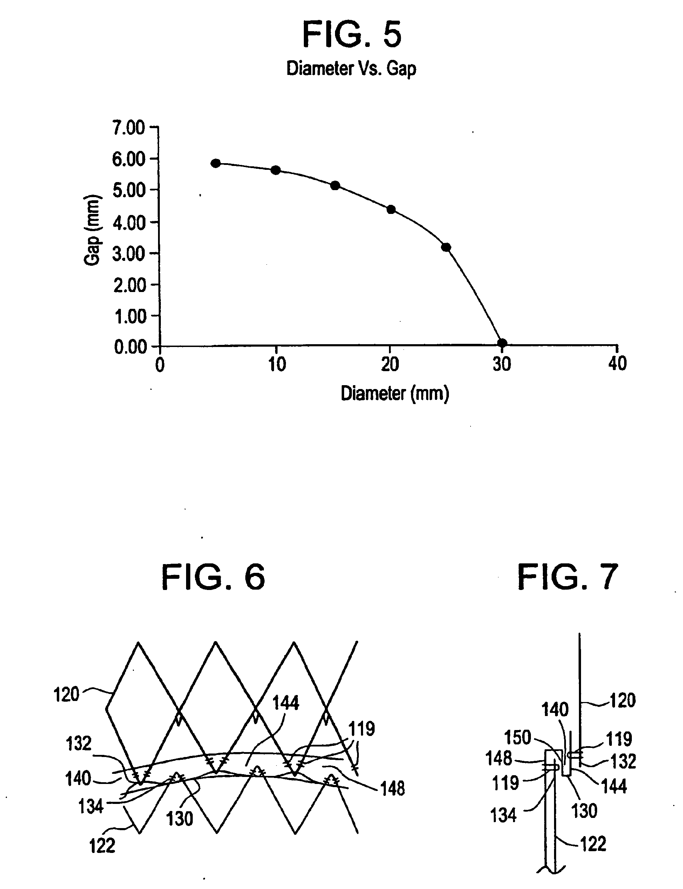

Fig. 5 illustrates the change in gap length as the diameter of the stents are increased in diameter from a crimped state ($D_1$) to an expanded state ($D_2$). For a device having a 30 mm expanded diameter (i.e., an endoprosthesis sized for treatment of an abdominal aortic ancurism), the plotted points can be curve fit to the following equation:

$$Gap(y) = \sqrt{34.7 - (\frac{diameter(\theta) * \pi}{16})^2} \ .$$

where the Gap(y) is the gap length between the lower apexes of the supra renal and upper apexes of the upper sealing stent, and the diameter(Q) is the diameter of such respective stents.

By decreasing the minimum sealing length, the outward force of the metal stent material of the supra renal and sealing stents 120, 122, 124 is maximized in the region most subject to leakage. In addition, the endoprosthesis of the invention has high flexibility given that the tether 130 is free to move between the apexes 132, 134 of stents 120, 122. Such freedom of motion allows the main body portion 112 of the endoprosthesis 110 to maintain flexibility across the minimum sealing length 136.

Referring to Figs. 2 through 4, 6 and 7, according to another aspect of the invention, and as previously mentioned, additional graft material is heat set into a pleat 140 at the top of the graft 118. The lower apex 132 of the supra renal stent 120 is stitched to an inner fold material 144 of the pleat 140, whereas the upper apex 134 of the upper sealing stent 122 is stitched to an outer fold material 148 of the pleat. As such, pleat material 150 is interposed between the respective lower and upper apexes 132, 134 of the supra renal and upper sealing stent 120, 122. The tether 130 is thread in and out of this pleat 140 as it is extends through the apexes of the supra renal and upper sealing stents. When the endoprosthesis 110 is crimped, the pleat 140 is preferably at least partially unfolded to accommodate stent separation. After expansion of the endoprosthesis, the heat set of the pleat 140 results in at least partial automatic re-gathering of the material into the pleat. The additional graft material at the pleat 140 provides multiple layers of graft material to increase bulk of the graft material at the location thereof and tissue contact thereat. This increased thickness of graft material provides better sealing at a critical location, and such double layer of graft material at the pleat 140 is contacted against the body tissue without any gap (or any significant gap) between the supra renal and upper sealing stents 120, 122.

According to another aspect of the invention, after implantation, the loading on the supra renal and sealing stents 120, 122 is shared between the tether 130 and the stitches 119 that couple the stents to the graft material 118. Such stitches 119 are preferably at or adjacent the apexes of the stents and/or along the struts of the stent. Initially substantially all the downward force of blood flow through the endoprosthesis (particularly as the endoprosthesis narrows into the legs 114, 116) is carried by the tether 130 rather than on the stitches that operate to attach the graft material 118 to the stents 120, 122. Upon being subject to such force, the tether is tensioned and loaded incrementally. If the tether is fully loaded, the pleat 140 in the graft material 118 then absorbs force and may partially unfold.

Only thereafter is the stitching 119 supporting the graft material 118 to the stents 120, 122 loaded and subject to force.

The endoprosthesis of the invention has high flexibility because the tether is free to move between the apexes of the stents. Such freedom of motion allows the main body portion of the endoprosthesis to main flexibility across the minimum sealing length.

There have been described and illustrated herein embodiments of an endoluminal prosthesis, particularly suitable for an AAA device. However, it is not intended that the invention be limited thereto. Thus, the tether and pleat may be used on endoluminal prostheses having no branches and/or intended for other purposes. In addition, while shape memory alloys, and preferably Nitinol have been disclosed as preferred materials for use in practicing the invention, it will be understood that other shape memory alloys and other shape set materials including biocompatible plastics may be used as well. Also, while the preferred graft material is the synthetic material polyethylene terephthalate, other graft materials including synthetic and natural materials can be used.

**Claims**

1. An endoluminal prosthesis (110), comprising:

   a tubular graft comprising a graft material (118):

      a first stent (120) including a first plurality of apexes (132); and
      a second stent (122) including a second plurality of apexes (134),
      a tether (130) extending through and freely movably relative to said first and second plurality of apexes (132, 134),

   wherein when said first and second stents (120, 122) are crimped to a first diameter slack in said tether (130) permits the first and second stents (120, 122) to be longitudinally displaced to provide a gap therebetween and wherein when said first and second stents (120, 122) are expanded to a second diameter said tether draws said first and second stents (120, 122) together to reduce or eliminate said gap; **characterised in that** said graft material (118) includes a pleat (140) defining an inner fold material (144), an outer fold material (148), and an interposed material (150) between said inner and outer fold materials (144, 148), said pleat (140) increasing the thickness of said graft material (118) at the location of said pleat;
   wherein the first plurality of apexes are in contact with said inner fold material and the second plurality of apexes are in contact with said outer fold material;
   wherein said pleat (140) can be at least partially unfolded to accommodate stent (120, 122) separation.

2. An endoluminal prosthesis according to claim 1, wherein:

said pleat (140) is at an end of said tubular graft.

3. An endoluminal prosthesis according to claim 1, wherein:

said second stent (124) is coupled to an interior surface of said tubular graft.

4. An endoluminal prosthesis according to claim 1, wherein:

a portion of said first stent (120) is coupled to an interior surface of said tubular graft.

5. An endoluminal prosthesis according to claim 1, wherein:

said first stent (120) is coupled to said tubular graft at attachment sites and said prosthesis is adapted for sharing of the loading on said first stent between said tether (130) and said attachment sites when said prosthesis is loaded by the flow of blood therethrough.

6. An endoluminal prosthesis according to claim 5, wherein:

said attachment sites are loaded only after said pleat (140) has been longitudinally loaded.

7. An endoluminal prosthesis according to claim 1, further comprising:

two tubular leg portions (114, 116),

wherein said tubular graft includes first and second ends, with said first stent coupled to said first end and said two leg portions (114, 116) coupled to said second end so that said prosthesis assumes a Y-configuration.

8. An endoluminal prosthesis according to claim 1, wherein:

said first plurality of apexes (132) of said first stent (120) are coupled to said graft material (118) and said first stent (120) longitudinally extends out of said graft material (118).

9. An endoluminal prosthesis according to claim 8, wherein:

said first stent includes anchors (120a) adapted for anchoring said device to body tissue.

10. An endoluminal prosthesis according to claim 1, wherein:

said first and second plurality of apexes (132, 134) are coupled to an interior surface of said graft material (118).

11. An endoluminal prosthesis according to claim 1, wherein:

said first plurality of apexes (132) are arranged about points on a first circle, and said second plurality of apexes (134) are arranged about points on a second circle.

**Patentansprüche**

1. Endoluminalprothese (110), welche Folgendes umfasst:

ein röhrenförmiges Transplantat, welches ein Transplantatmaterial (118) umfasst; einen ersten Stent (120), welcher eine erste Mehrzahl von Apexes (132) aufweist; und
einen zweiten Stent (122), welcher eine zweite Mehrzahl von Apexes (134) aufweist, ein Bindungselement (130), welches sich durch die erste und zweite Mehrzahl von Apexes (132, 134) erstreckt und relativ zu diesen frei beweglich ist,

wobei ein Spiel in dem Bindungselement (130) dem ersten und dem zweiten Stent (120, 122) erlaubt, longitudinal verschoben zu werden, um eine Lücke dazwischen bereitzustellen, wenn der erste und der zweite Stent (120, 122) zu einem ersten Durchmesser gecrimpt sind, und wobei das Bindungselement den ersten und den zweiten Stent (120, 122) zusammenzieht, um die Lücke zu verringern oder zu eliminieren, wenn der erste und der zweite Stent (120, 122) zu einem zweiten Durchmesser expandiert sind; **gekennzeichnet dadurch, dass**

das Transplantatmaterial (118) eine Falte (140) aufweist, welche ein inneres Faltmaterial (144), ein äußeres Faltmaterial (148), und ein Zwischenmaterial (150) zwischen dem inneren und dem äußeren Faltmaterial (144, 148) definiert, wobei die Falte (140) die Dicke des Transplantatmaterials (118) an dem Ort der Falte erhöht;

wobei die erste Mehrzahl von Apexes in Kontakt mit dem inneren Faltmaterial ist und die zweite Mehrzahl von Apexes in Kontakt mit dem äußeren Faltmaterial ist,

wobei die Falte (140) zumindest teilweise auseinandergefaltet werden kann, um einer Trennung der Stents (120, 122) Platz zu bieten.

2. Endoluminalprothese nach Anspruch 1, wobei die Falte (140) an einem Ende des röhrenförmigen Transplantats ist.

3. Endoluminalprothese nach Anspruch 1, wobei der zweite Stent (124) an eine innenliegende Oberfläche des röhrenförmigen Transplantats gekoppelt ist.

4. Endoluminalprothese nach Anspruch 1, wobei ein Teilbereich des ersten Stents (120) an eine innenliegende Oberfläche des röhrenförmigen Transplantats gekoppelt ist.

5. Endoluminalprothese nach Anspruch 1, wobei der erste Stent (120) an Befestigungsorten an das röhrenförmige Transplantat gekoppelt ist, und die Prothese geeignet ist, um die Belastung auf dem ersten Stent (120) zwischen dem Bindungselement (130) und den Befestigungsorten zu verteilen, wenn die Prothese durch den Blutfluss durch sie belastet ist.

6. Endoluminalprothese nach Anspruch 5, wobei die Befestigungsorte nur belastet sind, nachdem die Falte (140) longitudinal belastet wurde.

7. Endoluminalprothese nach Anspruch 1, welche weiterhin Folgendes umfasst:

   zwei röhrenförmige Beinbereiche (114, 116),

   wobei das röhrenförmige Transplantat ein erstes und ein zweites Ende aufweist, wobei der erste Stent an das erste Ende gekoppelt ist, und die zwei Beinbereiche (114, 116) an das zweite Ende gekoppelt sind, sodass die Prothese eine Y-Form annimmt.

8. Endoluminalprothese nach Anspruch 1, wobei die erste Mehrzahl von Apexes (132) des ersten Stents (120) an das Transplantatmaterial (118) gekoppelt sind, und sich der erste Stent (120) longitudinal aus dem Transplantatmaterial (118) erstreckt.

9. Endoluminalprothese nach Anspruch 8, wobei der erste Stent Ankerelemente (120a) aufweist, die geeignet sind, um die Vorrichtung an Körpergewebe zu verankern.

10. Endoluminalprothese nach Anspruch 1, wobei die erste und die zweite Mehrzahl von Apexes (132, 134) an eine innenliegende Oberfläche des Transplantatmaterials (118) gekoppelt sind.

11. Endoluminalprothese nach Anspruch 1, wobei die erste Mehrzahl von Apexes (132) um Punkte auf einem ersten Kreis angeordnet sind, und die zweite Mehrzahl von Apexes (134) um Punkte auf einem zweiten Kreis angeordnet sind.

**Revendications**

1. Prothèse endoluminale (110), comprenant :

   un greffon tubulaire comprenant un matériau de greffe (118),
   un premier stent (120) comprenant une première pluralité d'apex (132) ; et

un deuxième stent (122) comprenant une deuxième pluralité d'apex (134),

une attache (130) s'étendant dans les première et deuxième pluralités d'apex (132, 134) et étant librement mobile par rapport à ceux-ci,

dans laquelle, lorsque lesdits premier et deuxième stents (120, 122) sont repliés en un premier diamètre, un écart dans ladite attache (130) permet aux premier et deuxième stents (120, 122) d'être déplacés longitudinalement pour constituer un espace entre eux, et dans laquelle, lorsque lesdits premier et deuxième stents (120, 122) sont déployés en un deuxième diamètre, ladite attache tire lesdits premier et deuxième stents (120, 122) conjointement pour réduire ou éliminer ledit espace ; **caractérisée en ce que** ledit matériau de greffe (118) comprend un pli (140) définissant un, matériau de pliage interne (144), un matériau de pliage externe (148) et un matériau interposé (150) entre lesdits matériaux de pliage interne et externe (144, 148), ledit pli (140) augmentant l'épaisseur dudit matériau de greffe (118) à l'emplacement dudit pli ;

la première pluralité d'apex étant en contact avec ledit matériau de pliage interne et la deuxième pluralité d'apex étant en contact avec ledit matériau de pliage externe ;

ledit pli (140) pouvant être au moins partiellement déplié pour recevoir une séparation de stent (120, 122).

2. Prothèse endoluminale selon la revendication 1, dans laquelle ledit pli (140) se trouve à une extrémité dudit greffon tubulaire.

3. Prothèse endoluminale selon la revendication 1, dans laquelle ledit deuxième stent (124) est couplé à une surface intérieure dudit greffon tubulaire.

4. Prothèse endoluminale selon la revendication 1, dans laquelle une partie dudit premier stent (120) est couplée à une surface intérieure dudit greffon tubulaire.

5. Prothèse endoluminale selon la revendication 1, dans laquelle ledit premier stent (120) est couplé audit greffon tubulaire au niveau de sites de fixation et ladite prothèse est adaptée pour partager la charge sur ledit premier stent entre ladite attache (130) et lesdits sites de fixation lorsque ladite prothèse est chargée par le flux de sang passant au travers de celle-ci.

6. Prothèse endoluminale selon la revendication 5, dans laquelle lesdits sites de fixation ne sont chargés qu'après que ledit pli (140) a été chargé longitudinalement.

7. Prothèse endoluminale selon la revendication 1, comprenant en outre :

deux parties de jambes tubulaires (114, 116),

ledit greffon tubulaire comprenant des première et deuxième extrémités, ledit premier stent étant couplé à ladite première extrémité et desdites deux parties de jambes (114, 116) étant couplées à ladite deuxième extrémité de sorte que ladite prothèse présente une configuration en Y.

8. Prothèse endoluminale selon la revendication 1, dans laquelle ladite première pluralité d'apex (132) dudit premier stent (120) est couplée audit matériau de greffe (118) et ledit premier stent (120) s'étend longitudinalement hors dudit matériau de greffe (118).

9. Prothèse endoluminale selon la revendication 8, dans laquelle ledit premier stent comprend des ancres (120a) adaptées pour ancrer ledit dispositif aux tissus corporels.

10. Prothèse endoluminale selon la revendication 1, dans laquelle lesdites première et deuxième pluralités d'apex (132, 134) sont couplées à une surface intérieure dudit matériau de greffe (118).

11. Prothèse endoluminale selon la revendication 1, dans laquelle ladite première pluralité d'apex (132) est disposée autour de points sur un premier cercle, et ladite deuxième pluralité d'apex (134) est disposée sur un deuxième cercle.

# FIG. 1

## FIG. 2

## FIG. 3

## FIG. 4

# FIG. 5

### Diameter Vs. Gap

# FIG. 6

# FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9621404 A